# EUROPEAN PATENT APPLICATION

(11) **EP 2 433 997 A1**
(43) Date of publication of application: **28.03.2012**
(21) Application number: 10777709.6
(22) Date of filing: 14.05.2010
(51) Int. Cl.: C09K 3/00, A61K 8/02, A61K 8/44

(54) **LONG-CHAIN GLYCYL POLYOL TYPE GELLING AGENT AND GEL**

(30) Priority: 19.05.2009 JP 2009120852
(71) Applicant: Nissan Chemical Industries, Ltd., Chiyoda-ku Tokyo 101-0054 (JP); Kyushu University, Fukuoka-shi, Fukuoka 812-8581 (JP)
(72) Inventor: GOTO, Masahiro, Fukuoka-shi Fukuoka 812-8581 (JP); MARUYAMA, Tatsuo, Kobe-shi Hyogo 658-0051 (JP); MIYACHI, Nobuhide, Tokyo 101-0054 (JP)
(74) Representative: HOFFMANN EITLE
(86) International application number: PCT/JP2010/058210
(87) International publication number: WO 2010/134476

(57) **Abstract**

There is provided a gelator made of an aliphatic oxyglycyl polyol that is capable of forming a gel by a small amount of addition in a pH range from acidic to alkaline regions, and a gel having high environmental compatibility, biocompatibility and biodegradability. A gelator comprising an aliphatic oxyglycyl polyol of Formula (1) wherein R is a C₁₈₋₂₀ saturated aliphatic group or a C₁₈₋₂₀ unsaturated aliphatic group having one double bond) or a pharmaceutically usable salt thereof; a self-assembly formed by self-assembling of the gelator; and a gel comprising the gelator or the self-assembly, and water, an alcohol, an aqueous solution, an alcohol solution, a hydrophilic organic solution, or a hydrophobic organic solution.

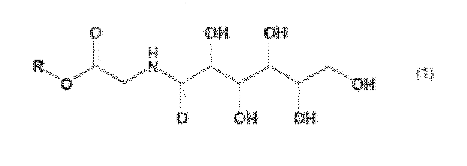

## Description

### TECHNICAL FIELD

The present invention relates to a novel aliphatic oxyglycyl polyol type gelator that can be easily produced in an industrial scale, a self-assembly that is formed by self-assembling of the gelator, and a gel that is composed of the gelator or the self-assembly and various types of aqueous solutions.
The aliphatic oxyglycyl polyol type gelator of the present invention is a gelator that can be easily synthesized by twice-reflux reaction and can be suitably used for the production of various gel bases for cosmetics, gel foods such as agar, pharmaceutical products, and the like. Furthermore, a gel obtained from the gelator is suitably used as various functional materials, for example, for commodities such as cosmetics, (soft) contact lenses, disposable diapers, and air fresheners, for dryland farming applications, for analytical chemistries such as chromatography, for medical and pharmaceutical applications, for biochemical fields such as protein carriers, cell culture related base materials, and bioreactors.

### BACKGROUND ART

A hydrogel is useful as a gel having high biocompatibility because it includes water as a medium, and thus is used in wide fields including commodities such as disposable diapers, cosmetics, and air fresheners.
Examples of conventional hydrogels include natural polymer gels such as agarose and synthetic polymer gels having cross-linkages between polymer chains through chemical covalent bonds, such as an acrylamide gel.

Recently, to such a hydrogel, various functions such as substance holding capacities, responsiveness to external stimulus, and biodegradability in consideration of the environment are imparted to form functional gels that have been attracting much attention, and there have been attempts for providing various functions by incorporating functional molecules into the natural or the synthetic polymer gels through copolymerization reaction or the like.

In order to impart a new function to a hydrogel in such a manner, it is required to study the nanostructure and the surface structure of the gel in detail. However, the method of incorporating a functional molecule through the copolymerization reaction has various problems that introduction ratio of a functional group is limited, that precise molecular design is difficult, that unreacted residual substances have safety issues, and that gel preparation is extremely complicated.

In contrast to such conventional "top-down type" developments of functional materials, "bottom-up type" studies for producing functional materials, in which atoms or molecules as a minimum unit of a substance are assembled to form an assembly as a supermolecule that provides a new function, has been drawing attention.
Also in the field of gels, there has been developed a new gel composed of a noncovalent gel fiber (so-called "nanofiber self-assembly") by self-assembling of a low-molecular weight compound. The "self-assembling" means that, in a substance (molecule) group in a random state at first, molecules are spontaneously assembled in a suitable external condition through intermolecular noncovalent interactions and the like to grow to a macro functional assembly.
The new gel draws attention because molecular design of a monomer achieves the control of intermolecular interactions or weak noncovalent bonds in a molecular assembly and consequently can theoretically control a macroscopic structure or a function of the gel.
However, there is no definite method for controlling the intermolecular interactions or the noncovalent bonds between low-molecular weight compounds. Furthermore, in the studies of the noncovalent gel, the study of self-assembly using hydrogen bonds in an organic solvent is advanced because the gel is comparatively readily formed, and self-assembled compounds in an aqueous solution (that is, a hydrogelatar and the like) have been found only incidentally.

Previously reported hydrogelators forming noncovalent gels are generally classified into the following three types.

### [1. Hydrogelators Having Amphiphilic Low-Molecular Weight Molecule as Skeleton]

This type of hydrogelators is modeled on an artificial lipid membrane. Examples of the hydrogelator include surfactant-type gelators having a quaternary ammonium salt part as the hydrophilic moiety and an alkyl long chain as the hydrophobic moiety and amphoteric surfactant-type gelators having two surfactant molecules that are connected to each other through the hydrophilic moiety.
As an example of the hydrogel produced by such gelators, there has been developed a molecular assembled hydrogel that is formed by adding an anionic compound having a molecular weight of 90 or more to an aqueous solution dispersing a cationic amphiphilic compound having a branched alkyl group as the hydrophobic moiety (Patent Document 1).

### [2. Hydrogelators Having Skeleton in Motif of Biocomponents]

Examples of the hydrogelators include gelators using association between molecular assemblies by a peptide secondary structure skeleton (such as an α-helix structure and a β-sheet structure).
For example, there have been developed a gelator having an α-helix structure (Non-Patent Document 1) and a gelator having a β-sheet structure (Non-Patent Document 2).

### [3. Hydrogelators Having Semi-Artificial Low-Molecular Weight Molecule as Skeleton]

This type of hydrogelators is composed of a combination of a biocomponent such as DNA bases, peptide chains, and sugar chains (hydrophilic moiety), an alkyl chain (hydrophobic moiety), and the like, and can be considered as a gelator that combines the characteristics of the above two types of gelators. Here, the DNA bases, the peptide chains, and the sugar chains have roles not only for improving hydrophilicity but also for imparting intermolecular interactions such as a hydrogen bond.
For example, there have been developed a hydrogelator composed of a glycoside-amino acid derivative including a sugar structure moiety having a glycoside structure of N-acetylated monosaccharides or disaccharides (Patent Document 2) and a fine hollow fiber composed of a peptide lipid of General Formula "RCO(NHCH₂CO)mOH" and a transition metal and having self-assembling properties (Patent Document 3).
There is also disclosed a formation of β-sheet fiber network from an amphiphilic peptide having a structure of<hydrophobic moiety-cysteine residue (forming disulfide bonds at the time of network formation)-glycine residue (imparting flexibility)-phosphorylated serine residue-cell adhesive peptide> using the hydrophobic moiety as a core (Non-Patent Document 3).
In addition, there has been reported a preparation of a glycolipid supermolecular hydrogel using a chemical library (Non-Patent Document 4).

Amphiphilic dipeptide compounds composed of a hydrophobic moiety and a dipeptide are also drawing attention as one of the "bottom-up type" functional materials capable of forming a self-assembly. For example, it is known that a dipeptide compound having a specific lipid moiety of "2-(naphthalen-2-yloxy)acetic acid" and "glycylglycine, glycylserine, or the like" can form a hydrogel. However, each can form a gel with an acidic aqueous solution, or a hydrogel formed from each is merely acidic (Non-Patent Document 5).
In contrast, a lipid peptide compound composed of lauric acid or myristic acid that is a naturally occurring fatty acid and glycylglycine does not form a hydrogel but forms an organic nanotube including multilayered vesicles and a hollow having an inner diameter of about 50 to 90 nm to be precipitated (for example, Patent Document 3).
Furthermore, among the amphiphilic compounds, a lipid glycyl polyol is applied as a surfactant or an emulsifier (Non-Patent Document 6). However, self-assemblies formed by self-assembling of lipid glycyl polyols (1a to 3a) described in the document cannot form hydrogels.

### Related Art Documents

### Patent Documents

Patent Document 1: Japanese Patent Application Publication No. JP-A-2002-085957
Patent Document 2: Japanese Patent Application Publication No. JP-A-2003-327949
Patent Document 3: Japanese Patent Application Publication No. JP-A-2004-250797

### Non-Patent Documents

Non-Patent Document 1: W.A. Pekata et. al., SCIENCE, 281, 389 (1998)
Non-Patent Document 2: A. Aggeli et. al., Angew. Chem. Int. Ed., 2003, 42, 5603-5606
Non-Patent Document 3: Jeffry D. Hartgerink, Elia Beniaah, Samuel I. Stupp, SCIENCE, vol 294, 1684-1688 (2001)
Non-Patent Document 4: Shinji Matsumoto, Itaru Hamachi, Dojin News, No. 118, 1-16(2006)
Non-Patent Document 5: Z. Yang, B. Xu et.al., J. Mater. Chem., 2007, 17, 850-854
Non-Patent Document 6: M. Suzuki, S. Owa, H. Shirai, and K. Hanabusa, Tetrahedron, 63 (2007) 7302-7308

### DISCLOSURE OF THE INVENTION

### Problem to be Solved by the Invention

In conventional hydrogels, for forming a synthetic polymer gel, or sometimes for forming a gel from a natural polymer such as gelatin (collagen), a crosslinking agent having an aldehyde group is required to be used.
Furthermore, in order to impart a function to a natural polymer gel as well as a (synthetic) polymer gel, chemical modification of the polymer chain or copolymerization reaction for incorporating a functional molecule is required.
In this manner, conventional hydrogels have problems that gel preparation is complicated and that unreacted crosslinking agents or unreacted substances during copolymerization reaction remain in a hydrogel.

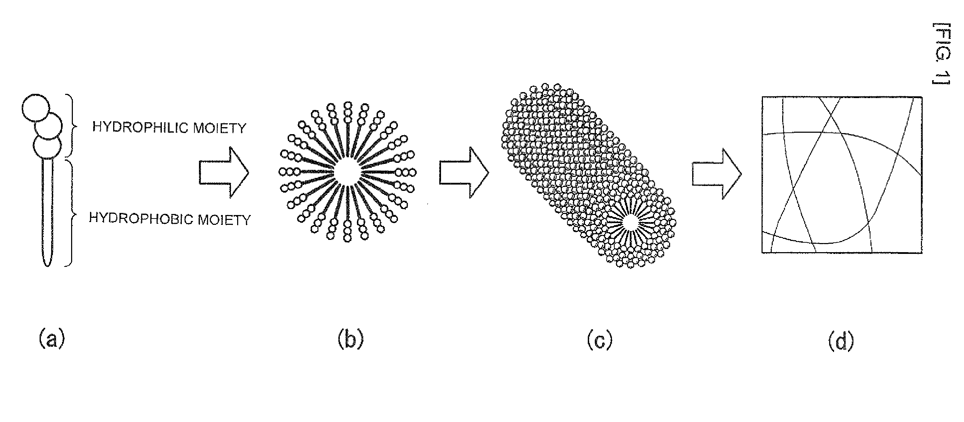

Furthermore, among previously developed hydrogelators forming noncovalent gels, the hydrogelator having the amphiphilic low-molecular weight molecule as the skeleton (1.) may not form a gel depending on the pH of a medium. That is, in an alkaline region, the gelator forms micelles to lead to an emulsion. In contrast, in an acidic region, the gelator is fibrously self-assembled to form a hydrogel. However, in a neutral region that is considered to be safe for living bodies, there are few reports on hydrogelation. Furthermore, there is a problem that quaternary ammonium cations and the like (for example Patent Document 1) may have safety issues in a biological environment.
The hydrogelator having a skeleton in the motif of biocomponents (2.) has a problem of productivity, that is, unsuitable for mass production, and a problem that gel forming ability are affected by temperature or pH.
In addition, the hydrogelator having a semi-artificial, low-molecular weight molecule as the skeleton (3.) also has problems in biocompatibility and environmental safety. For example, according to Patent Document 2, the use of highly toxic sodium azide is shown in a reaction scheme (FIG. 1) for synthesizing a glycoside-amino acid derivative constituting the hydrogelator. According to Patent Document 3, a transition metal (ion) is required to be added for self-assembling of the hollow fiber.

In this manner, in previously reported various noncovalent hydrogels and hydrogelators for forming the gels, there is a demand for further improving the gel forming ability (gel structure holding ability), the safety in a biological environment, and the like.
From the viewpoint of the safety in the biological environment, there is a potential demand of a hydrogelator capable of forming a gel by a smaller amount of addition.

In view of the above, it is an object of the present invention to provide a gelator composed of a lipid glycine polyol that is produced using a safe and highly versatile higher alcohol usable for cosmetics and medical preparations, a naturally occurring amino acid, and a polyol, that can be easily obtained at low cost by two-step reaction through two types of reflux reaction, which enables industrial scale production, and that has high gel forming ability capable of forming a gel even by a small amount of addition.
In particular, the present invention has an object to provide a gelator having high gel forming ability capable of forming a gel by an extremely small amount of addition of an aqueous mixed solution of an alcohol or an organic solvent and an aqueous solution dissolving an inorganic salt or an organic salt, in a wide pH range from acidic to alkaline regions, specifically even in a neutral region.
The present invention further has an object to provide a gel that keeps a stable gel structure in a wide pH range from acidic to alkaline regions and that has high environmental compatibility, biocompatibility, and biodegradability.

### Means for Solving the Problem

As a first aspect, the present invention relates to a gelator characterized by including an aliphatic oxyglycyl polyol of Formula (1):

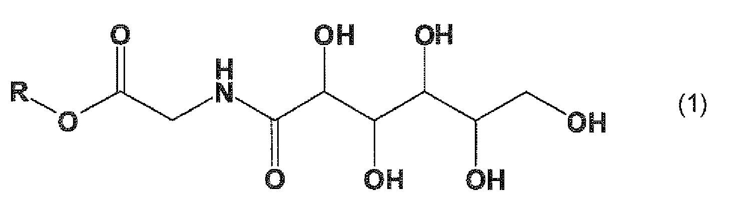

(where R is a C₁₈₋₂₀ saturated aliphatic group or a C₁₈₋₂₀ unsaturated aliphatic group having one double bond) or a pharmaceutically usable salt thereof.
As a second aspect, the present invention relates to the gelator according to the first aspect, in which R is a C₁₈ saturated aliphatic group or a C₁₈ unsaturated aliphatic group having one double bond.
As a third aspect, the present invention relates to the gelator according to the first aspect, in which R is a stearyl group or an oleyl group.
As a fourth aspect, the present invention relates to a self-assembly formed by self-assembling of the gelator as described in any one of the first aspect to the third aspect.
As a fifth aspect, the present invention relates to a gel including the gelator as described in any one of the first aspect to the third aspect or the self-assembly as described in the fourth aspect; and water, an alcohol, an aqueous solution, an alcohol solution, a hydrophilic organic solution, or a hydrophobic organic solution.
As a sixth aspect, the present invention relates to the gel according to the fifth aspect, in which the alcohol solution is an aqueous solution of at least one alcohol selected from a group consisting of methanol, ethanol, 2-propanol, and i-butanol.
As a seventh aspect, the present invention relates to the gel according to the fifth aspect, in which the hydrophilic organic solution is a solution of at least one hydrophilic organic solvent selected from a group consisting of acetone and dioxane.
As an eighth aspect, the present invention relates to the gel according to the fifth aspect, in which the aqueous solution is an aqueous solution dissolving at least one inorganic salt selected from a group consisting of inorganic carbonates, inorganic sulfates, inorganic phosphates, and inorganic hydrogen phosphates or at least one organic salt selected from a group consisting of organic amine hydrochlorides and organic amine acetates.
As a ninth aspect, the present invention relates to the gel according to the eighth aspect, in which the inorganic salt is at least one inorganic salt selected from a group consisting of calcium carbonate, sodium carbonate, potassium carbonate, sodium sulfate, potassium sulfate, magnesium sulfate, potassium phosphate, sodium phosphate, disodium hydrogen phosphate, and sodium dihydrogen phosphate, and the organic salt is at least one organic salt selected from a group consisting of ethylenediamine hydrochloride, ethylenediaminetetraacetate, and tris(hydroxymethyl)aminomethane hydrochloride.
As a tenth aspect, the present invention relates to the gel according to the fifth aspect, in which the hydrophobic organic solution is a solution of at least one hydrophobic organic solvent selected from a group consisting of vegetable oils, esters, and hydrocarbons.

### Effects of the Invention

The gelator of the present invention can form a gel by gelation of an aqueous medium such as water, an alcohol, an aqueous solution, an alcohol solution, and a hydrophilic organic solution without using a crosslinking agent or the like that is required for conventional gel formation, and thus an unreacted crosslinking agent does not remain. Furthermore, the gelator of the present invention is composed of a low-molecular weight compound, and thus can form a gel without containing unreacted substances of functional molecules which are incorporated for providing functions, in a same manner as conventional gelators.
Moreover, the gelator of the present invention can form a gel with not only the aqueous medium but also a hydrophobic medium such as a hydrophobic organic solution containing oil and the like.

Unlike conventional low-molecular weight gelators, the gelator of the present invention is composed of a higher alcohol that can be used as the additive for cosmetics and pharmaceutical products, a polyol, and naturally occurring glycine, and thus has high biological safety. Moreover, it can be synthesized more easily and in a larger amount by twice-reflux reaction. Hence, it is a low-molecular weight gelator excellent in economy.

The gelator of the present invention can also form a gel in a wide pH range from an acidic region to an alkaline region even with an alcohol solution, an aqueous solution mixed with a hydrophilic organic solvent, and an aqueous solution dissolving an inorganic salt or an organic salt. In particular, from the viewpoint of high safety that is required in cell culture substrates, medical materials, cosmetic materials, and the like, the gelator of the present invention is very useful for such applications because it has gel forming ability for various aqueous solutions in a neutral region.

The gelator of the present invention can have gel forming ability as a gelator even when two or more of aliphatic oxyglycyl polyols constituting the gelator are mixed.
Furthermore, even when the gelator of the present invention is mixed with other various peptides capable of forming a self-assembly, that is, tripeptides or tetrapeptides having the N-terminal modified with a fatty acid, in addition to the aliphatic oxyglycyl polyol of Formula (1), each self-assembly or a mixed self-assembly can be formed.
Moreover, the gelator of the present invention can form a self-assembly even with an aqueous solution dissolving an anionic surfactant, a nonionic surfactant, or a cationic surfactant, while mixing the surfactant.

The gelator of the present invention can be self-assembled to form a self-assembly that adsorbs or includes a low-molecular weight compound, and consequently can form a gel capable of sustained-releasing the low-molecular weight compound.

The gelator of the present invention does not use animal-derived materials (such as collagen, gelatin, and matrigel) that recently have the issue of BSE infection and the like but is an artificial low-molecular weight compound composed of a lipid and a peptide alone. Thus, the obtained gel has no issue caused by such infection and the like. Moreover, the gelator can be produced only by amidation of a lipid and a peptide without using a toxic reagent with high reactivity, such as sodium azide, and thus can be suitably used as a highly safe gelator.
The gelator of the present invention can also be used as a cellular damage protection material and Langmuir monolayer along with the applications as a gel.

The self-assembly of the present invention has the outermost side (that is, the self-assembly surface) on which the polyol moiety is located when the gelator is self-assembled while placing the hydrophobic group at the center. Hence, it less causes rejection of living cells and has excellent cell-adhesive properties when it is incorporated in a living body. On this account, the self-assembly is preferably used for sustained-release carriers and adsorbents for medical use, scaffolding materials for regenerative medicine, and the like.
In addition to the above applications, the self-assembly is useful as stabilizers, dispersants, and wetting agents in food industries, agriculture and forestry, cosmetic fields, and fiber industries, as nano components doped with a metal or an electrically-conductive substance in electronics and information fields, and as filter materials and electrically-conductive materials.

The gel of the present invention is preferably used as biochemical materials and medical materials for cell culture and the like because it can stably keep a gel structure in a wide pH range from an acidic region to an alkaline region, especially in a neutral condition.
Furthermore, the gel of the present invention is a highly safe gel in both biological aspects and environmental aspects because it can be obtained by addition of the gelator in a smaller amount than that of conventional gelators as described above.
Moreover, as described above, the gel obtained from an aliphatic oxyglycyl polyol as a low-molecular weight compound reduces environmental and biological burdens because it can be readily decomposed by soil bacteria and the like when it is used in an external environment, for example, in soil, and because it can be readily decomposed by metabolic enzymes when it is used in a living body.

### BEST MODES FOR CARRYING OUT THE INVENTION

### [Gelator]

The gelator of the present invention is composed of an aliphatic oxyglycyl polyol having a structure of Formula (1) below or a pharmaceutically usable salt thereof. The aliphatic oxyglycyl polyol is composed of a higher alcohol-derived moiety having a highly lipophilic long chain, a glycine-derived moiety, and a polyol-derived (gluconolactone-derived) moiety.

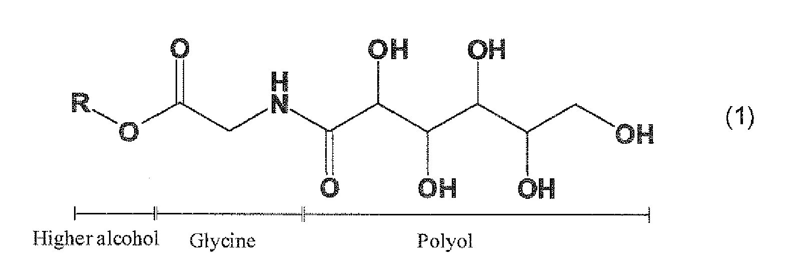

In Formula (1), R included in the higher alcohol-derived moiety is a C₁₈₋₂₀ saturated aliphatic group or a C₁₈₋₂₀ unsaturated aliphatic group having one double bond.
Preferably, R is a C₁₈ saturated aliphatic group or a C₁₈ unsaturated aliphatic group having one double bond, and is specifically preferably a stearyl group (octadecyl group) or an oleyl group.

### [Self-Assembly Formed From Gelator]

When the gelator of the present invention is poured into water, an alcohol, an aqueous solution, an alcohol solution, or a hydrophilic organic solution, the glycine-derived moiety and the polyol-derived moiety in Formula (1) form intermolecular noncovalent bonds through hydrogen bonds, while the higher alcohol-derived moiety in Formula (1) is hydrophobically packed to be self-assembled (also referred to as self-organized), and consequently a self-assembly is formed.
As reference, FIG. 1 shows an exemplified schematic diagram of self-assembling and gelation of the aliphatic oxyglycyl polyol constituting the gelator of the present invention (in the invention, not all of the aliphatic oxyglycyl polyols form the self-assembly or the gel shown in FIG. 1).
The aliphatic oxyglycyl polyol molecules (a) are assembled to place the higher alcohol-derived moieties as the hydrophobic moiety at the center (b) and self-assembled to form a self-assembly (c). The self-assembly may have any shape, and examples of the shape include a tubular shape and a plate-like shape.
When the gelator of the present invention is poured into a hydrophobic organic solution such as a vegetable oil, the glycine-derived moiety and the polyol-derived moiety in Formula (1) are conversely hydrophilically packed to be self-assembled, and a self-assembly is formed.

### [Gel]

When the self-assembly is formed in an aqueous medium such as water, an alcohol, an aqueous solution, an alcohol solution, and a hydrophilic organic solution, the self-assembly forms a three-dimensional network structure (for example, see FIG. 1 (d)). Then, the hydrophilic moiety (the glycine-derived moiety and the polyol-derived moiety) on the surface of the self-assembly forms noncovalent bonds with the aqueous medium to be swelled, and the gelation of the aqueous medium proceeds to form a hydrogel.
When the self-assembly is formed in a hydrophobic medium such as a vegetable oil (a hydrophobic organic solution), the self-assembly similarly forms a three-dimensional network structure. Then, the hydrophobic moiety (the higher alcohol-derived moiety) on the surface of the self-assembly and the hydrophobic medium are assembled through hydrophobic interactions, and the gelation of the hydrophobic medium proceeds to form a gel.

The aqueous medium is not specifically limited as long as a medium does not interfere with the self-assembling or gelation of the gelator. Specific usable examples of the preferred aqueous medium include water, an aqueous solution dissolving an inorganic salt or an organic salt in water (referred to as an aqueous solution in the present specification), an alcohol, a mixed solution of water and an alcohol (referred to as an alcohol solution in the present specification), and a mixed solution of water and a hydrophilic organic solvent (referred to as a hydrophilic organic solution in the present specification).

The alcohol is preferably a water-soluble alcohol freely dissolved in water, and more preferably C₁₋₆ alcohols, polyhydric alcohols, higher alcohols, and glycerides.
Specifically, examples of the C₁₋₆ alcohol include methanol, ethanol, 2-propanol, and i-butanol; examples of the polyhydric alcohol include ethylene glycol, propylene glycol, and polypropylene glycol; examples of the higher alcohol include octyldodecanol, stearyl alcohol, and oleyl alcohol; and examples of the glyceride include trioctanoin, glyceryl tri(caprylate/caprate), and glyceryl stearate.
The hydrophilic organic solvent is an organic solvent other than alcohols and means an organic solvent dissolved in water at any ratio. Examples of the hydrophilic organic solvent to be used include acetone and dioxane.

A plurality of the inorganic salts or the organic salts may be added but one or two salts are preferably added. It is desirable that two salts be added to provide buffering capacity to a solution.
Preferred examples of the inorganic salt include inorganic carbonates, inorganic sulfates, inorganic phosphates, and inorganic hydrogen phosphates. More preferred are calcium carbonate, sodium carbonate, potassium carbonate, sodium sulfate, potassium sulfate, magnesium sulfate, potassium phosphate, sodium phosphate, disodium hydrogen phosphate, and sodium dihydrogen phosphate, and even more preferred are calcium carbonate, magnesium sulfate, disodium hydrogen phosphate, and sodium dihydrogen phosphate.
Preferred examples of the organic salt include hydrochlorides of organic amines and acetates of organic amines. More preferred are ethylenediamine hydrochloride, ethylenediaminetetraacetate, and tris(hydroxymethyl)aminomethane hydrochloride.

The hydrophobic medium is not specifically limited as long as a medium does not interfere with the self-assembling or gelation of the gelator. As a preferred specific example, a solution of at least one hydrophobic organic solvent selected from a group consisting of vegetable oils, esters, and hydrocarbons may be used.
Specifically, preferred examples of the hydrophobic medium include vegetable oils such as olive oil, coconut oil, castor oil, jojoba oil, and sunflower oil; esters such as cetyl octanoate, isopropyl myristate, and isopropyl palmitate; and hydrocarbons such as mineral oil and hydrogenated polyisobutene.
In the present invention, these hydrophobic mediums are collectively referred to as the hydrophobic organic solution.

Mechanisms of the hydrogel formation when the gelator of the present invention is poured into an aqueous medium are supposed to be attributed by those described below.
Namely, in the hydroxy group moiety (the polyol-derived moiety) of the aliphatic oxyglycyl polyol constituting the gelator, the hydrogen atoms are not dissociated in conditions ranging from acidic to neutral regions, and the hydroxy groups form hydrogen bonds to each other for the self-assembling. Meanwhile in an alkaline region, the hydrogen atoms are dissociated from the hydroxy group moiety, to which metal ions present in the solution are bonded. Consequently, cross-linkages are formed through the metal ions for the self-assembling.

As described above, the gelator of the present invention can form a stable gel even in a neutral region. Furthermore, the gelator is composed of an aliphatic oxyglycyl polyol that is a low-molecular weight compound derived from natural substances and safe substances as materials, and hence the gelator and the gel obtained from the gelator can be degraded in environments and living bodies. Therefore, a gelator and a gel having high biocompatibility can be obtained.

On this account, the gelator of the present invention and the gel obtained from the gelator can be used as materials in various fields such as cell culture substrates, storage materials for biomolecules such as cells and proteins, base materials for external preparations, materials for medical use, biochemical materials, cosmetic materials, food materials, contact lenses, disposable diapers, artificial actuators, dryland farming materials. Furthermore, they can be widely used as bioreactor carriers for enzymes and the like for researches, medical cares, analyses, and various industries.

The gel of the present invention is a gel formed from a low-molecular weight compound (aliphatic oxyglycyl polyol). Thus, designing of the compound can readily impart various functions without polymer chain modification or copolymerization reaction. For example, a gel capable of being transformed between sol and gel by external stimulus response can be formed.

### Examples

Hereinafter, the present invention will be described in further detail with reference to examples, but the present invention is not limited to these examples.

### [Abbreviations Used in Examples]

Abbreviations used in the examples below mean the following compounds.
Gly: glycine
Ala: alanine

### [Synthesis of Long Chain Glycol Polyol]

### Synthetic Example 1: Synthesis of Long Chain Glycyl Polyol (Aliphatic Oxyglycyl Polyol) of Formula (1)

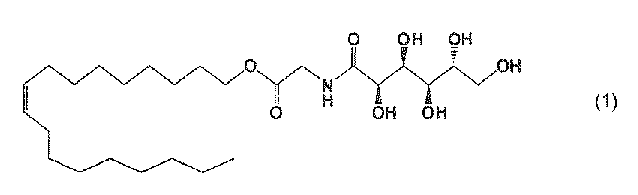

Oleyl alcohol (20.0 g, 74.5 mmol) and glycine (6.2 g, 82.6 mmol) were suspended in toluene (400 mL), then p-toluenesulfonic acid monohydrate (23.6 g, 124 mmol) was added, and the whole was heated and refluxed for 4 hours. After cooling, the reaction mixture was concentrated under reduced pressure to a half or less, and diluted with methylene chloride. Then, the solution was successively washed with a saturated aqueous sodium hydrogen carbonate solution and a saturated salt solution.
The organic phase was dried over sodium sulfate, then filtered, and concentrated under reduced pressure. The obtained residue was purified by silica gel column chromatography (silica gel 200 g, methanol : methylene chloride = 0:100 to 1:30) to give a target compound (17.9 g, 55.0 mmol, yield 67%) as a brown liquid.
The compound obtained in this manner (17.9 g, 55.0 mmol) was dissolved in ethanol (350 mL), then D-(+)-glucono-1,5-lactone (9.8 g, 55.0 mmol) was added, and the whole was heated and refluxed for 5 hours. After cooling, the reaction mixture was slowly stirred at room temperature overnight. The resulting crystal was filtered, then washed with methanol, and dried to give an aliphatic oxyglycyl polyol of Formula (1) (17.5 g, 34.7 mmol, yield 63%) as a white crystal.

¹H NMR (500 MHz, DMSO-d6, δ ppm): 7.99 (1H, t, J=5.8 Hz, ¹NH Gly), 5.49 (1H, d, J= 5.2 Hz, -OH), 5.4-5.3 (2H, m, -CH=CH-), 4.54 (1H, d, J=5.2 Hz, -OH), 4.47 (1H, d, J=5.8 Hz, -OH), 4.37 (1H, d, J=7.4 Hz, -OH), 4.34 (1H, t, J=5.5 Hz, -CH₂-OH), 4.08-4.01 (3H, m, -CH(OH)-, -CH₂O-), 3.94-3.89 (2H, m, -CH(OH)-, α-CH Gly), 3.77 (1H, m, α-CH Gly), 3.57 (1H, m, -CH₂-OH), 3.52-3.44 (2H, m, -CH(OH)- × 2), 3.38 (1H, m, -CH₂-OH), 2.0-1.9 (4H, m, -CH₂-CH=CH-CH₂-), 1.58-1.52 (2H, m, -CH₂-), 1.34-1.20 (22H, m, -CH₂-), 0.85 (3H, t, J=7.1 Hz, CH₃).
FT-MS +m/z calc. for C26H5008N1 [M+H]+ 504.35364, found 504.3541.

Synthetic Example 2: Synthesis of Long Chain Glycyl Polyol (Aliphatic Oxyglycyl Polyol) of Formula (2) 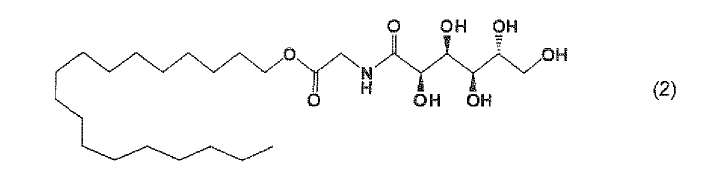 Octadecanol (7.9 g, 29.2 mmol) and glycine (2.0 g, 26.6 mmol) were suspended in toluene (80 mL), then p-toluenesulfonic acid monohydrate (6.6 g, 34.7 mmol) was added, and the whole was heated and refluxed for 18 hours. After cooling, the reaction mixture was diluted with methylene chloride, and successively washed with a saturated aqueous sodium hydrogen carbonate solution and a saturated salt solution. Next, the organic phase was dried over sodium sulfate, then filtered, and concentrated under reduced pressure. To the obtained residue, a 4M hydrochloric acid-dioxane solution (20 mL) was added, and concentrated under reduced pressure. Then, the resulting crystal was filtered, and washed with ethyl acetate. The crystal was dissolved in methylene chloride once again. To the solution, a saturated aqueous sodium hydrogen carbonate solution was added for separation. The separated organic phase was dried over sodium sulfate, then filtered, and concentrated under reduced pressure to give a target compound (5.5 g, 16.8 mmol), yield 63%) as a white solid.
The compound obtained as described-above (5.5 g, 16.8 mmol) was suspended in ethanol (100 mL), then D-(+)-glucono-1,5-lactone (3.0 g, 16.8 mmol) was added, and the whole was heated and refluxed for 5 hours. After cooling, the reaction mixture was slowly stirred at room temperature overnight. The resulting crystal was faltered, then washed with methylene chloride, and dried to give an aliphatic oxyglycyl polyol of Formula (2) (6.1 g, 12.1 mmol, yield 72%) as a white crystal.

¹H NMR (500 MHz, DMSO-d6, δ ppm): 7.92 (1H, t, J=5.8 Hz ¹NH Gly), 5.40 (1H, d, J=4.9 Hz, -OH), 4.46 (1H, d, J=5.2 Hz, -OH), 4.40 (1H, d, J=5.5 Hz, -OH), 4.30 (1H, d, J=7.0 Hz, -OH), 4.27 (1H, t, J=5.5 Hz, -CH₂-OH), 4.08-4.02 (3H, m, -CH(OH)-, -CH₂O-) 3.94-3.88 (2H, m, -CH(OH)-, α-CH Gly), 3.79 (1H, m, α-CH Gly), 3.58 (1H, m, -CH₂-OH), 3.52-3.44 (2H, m, -CH(OH)- × 2), 3.38 (1H, m, -CH₂-OH), 1.60-1.54 (2H, m, -CH₂-), 1.35-1.20 (30H, m, -CH₂-), 0.86 (3H, t, J=7.1 Hz, CH₃).
FT-MS +m/z calc. for C26H52O8N1 [M+H]+ 506.36929, found 506.3696.

Synthetic Example 3: Synthesis of Long Chain Glycyl Polyol of Formula (3) 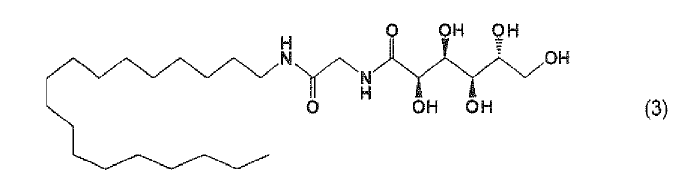 Stearylamine (200 mg, 0.74 mmol) and N-Boc-glycine (156 mg, 0.89 mmol) were suspended in methylene chloride (4 mL), then WSC (water-soluble carbodiimide: 185 mg, 0.96 mmol) and diisopropylethylamine (126 mL, 0.74 mmol) were added, and the whole was stirred for 4 hours. The reaction mixture was diluted with methylene chloride, and successively washed with a 1M aqueous hydrochloric acid solution, a saturated aqueous sodium hydrogen carbonate solution, and a saturated salt solution.
The organic phase was dried over sodium sulfate, then filtered, and concentrated under reduced pressure. The obtained residue was dissolved in methylene chloride (4 mL), then a 4M hydrochloric acid-dioxane solution (3 mL) was added, and the whole was stirred for 3 hours. The resulting crystal was filtered, and washed with methylene chloride. The obtained crystal was dissolved in methylene chloride once again. To the solution, a saturated aqueous sodium hydrogen carbonate solution was added for separation. The separated organic phase was dried over sodium sulfate, then filtered, and concentrated under reduced pressure to give a target compound (106 mg, 0.32 mmol, yield 43% for two steps) as a white crystal.
The compound obtained as described-above (100 mg, 0.31 mmol) was dissolved in ethanol (5 mL), then D-(+)-glucono-1,5-lactone (55 mg, 0.31 mmol) was added, and the whole was heated and refluxed for 3 hours. After cooling, the reaction mixture was slowly stirred at room temperature overnight. The resulting crystal was filtered, then washed with methanol, and dried to give a long chain glycyl polyol of Formula (3) (110 mg, 0.22 mmol, yield 70%) as a white crystal.

¹H NMR(500 MHz, DMSO-d6, δ ppm): 7.94 (1H, t, J=5.8 Hz, ¹NH Gly), 7.73 (1H, t, J=5.8 Hz, -NH-), 5.60 (1H, d, J=4.6 Hz, -OH), 4.63 (1H, d, J=6.2 Hz, -OH), 4.59 (1H, d, J=5.5 Hz, -OH), 4.54 (1H, d, J=7.0 Hz, -OH), 4.36 (1H, t, J=5.5 Hz, -CH₂-OH), 4.05 (1H, m, -CH(OH)-), 3.92 (1H, m, -CH(OH)-), 3.73 (1H, m, α-CH Gly), 3.64-3.42 (4H, m, α-CH Gly, -CH₂-OH, -CH(OH)- × 2), 3.37 (1H, m, -CH(OH)-), 3.10-2.96 (2H, m, -CH₂NHCO-), 1.42-1.32 (2H, m, -CH₂-), 1.30-1.20 (30H, m, -CH₂-), 0.85 (3H, t, J=7.1 Hz, CH₃).
FT-MS +m/z calc. for C26H53O7N2 [M+H]+ 505.38528, found 505.3856.

Synthetic Example 4: Synthesis of Long Chain Glycyl Polyol of Formula (4) 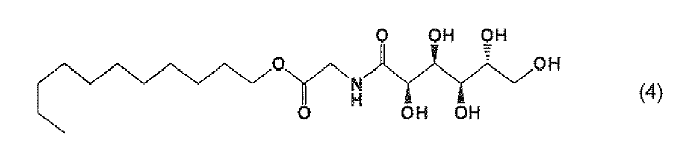 Undecanol (304 mL, 1.47 mmol) and glycine (100 mg, 1.33 mmol) were suspended in toluene (2 mL), then p-toluenesulfonic acid monohydrate (330 mg, 1.73 mmol) was added, and the whole was heated and refluxed for 3 hours. After cooling, the reaction mixture was diluted with methylene chloride, and successively washed with a saturated aqueous sodium hydrogen carbonate solution and a saturated salt solution. Next, the organic phase was dried over sodium sulfate, then filtered, and concentrated under reduced pressure. To the obtained residue, a 4M hydrochloric acid-dioxane solution (3 mL) was added, and concentrated under reduced pressure. Then, the resulting crystal was filtered, and washed with ethyl acetate. The crystal was dissolved in methylene chloride once again. To the solution, a saturated aqueous sodium hydrogen carbonate solution was added for separation. The separated organic phase was dried over sodium sulfate, then filtered, and concentrated under reduced pressure to give a target compound (259 mg, 1.13 mmol, yield 85%) as a pale yellow liquid.
The compound obtained as described-above (212 mg, 0.92 mmol) was dissolved in ethanol (4 mL), then D-(+)-glucono-1,5-lactone 14 (165 mg, 0.92 mmol) was added, and the whole was heated and refluxed for 3 hours. After cooling, the reaction mixture was slowly stirred at room temperature overnight. The resulting crystal was filtered, then washed with methylene chloride, and dried to give a long chain glycyl polyol of Formula (4) (268 mg, 0.66 mmol, yield 71 %) as a white crystal.

¹H NMR (500 MHz, DMSO-d6, δ ppm): 8.00 (1H, t, J=5.8 Hz, ¹NH Gly), 5.49 (1H, d, J=5.2 Hz, -OH), 4.54 (1H, d, J=5.2 Hz, -OH), 4.48 (1H, d, J=5.8 Hz, -OH), 4.38 (1H, d, J=7.3 Hz, -OH), 4.34 (1H, t, J=5.8 Hz, -CH₂-OH), 4.08-4.02 (3H, m, -CH(OH)-, -CH₂O-), 3.94-3.88 (2H, m, -CH(OH)-, α-CHGly), 3.77 (1H, m, α-CH Gly), 3.58 (1H, m, -CH₂-OH), 3.52-3.44 (2H, m, -CH(OH)- × 2), 3.38 (1H, m, -CH₂-OH), 1.60-1.54 (2H, m, -CH₂-), 1.35-1.20 (16H, m, -CH₂-), 0.86 (3H, t, J=7.1 Hz, CH₃).
FT-MS +m/z calc. for C19H38O8N1 [M+H]+ 408.25974, found 408.2589.

Synthetic Example 5 Synthesis of Long Chain Glycyl Polyol of Formula (5) 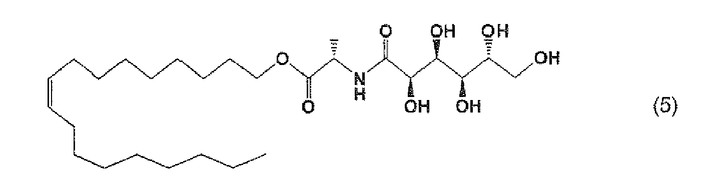 Oleyl alcohol (330 mg, 1.23 mmol) and L- alanine (100 mg, 1.12 mmol) were suspended in toluene (4 mL), then p-toluenesulfonic acid monohydrate (278 mg, 1.46 mmol) was added, and the whole was heated and refluxed for 3 hours. After cooling, the reaction mixture was diluted with methylene chloride, and successively washed with a saturated aqueous sodium hydrogen carbonate solution and a saturated salt solution. Next, the organic phase was dried over sodium sulfate, then filtered, and concentrated under reduced pressure. The obtained residue was purified by silica gel column chromatography (silica gel 2 g, methanol: methylene chloride = 0:100 to 1:30) to give a target compound (358 mg, 1.06 mmol), yield 95%) as a brown syrup.
The compound obtained in this manner (304 mg, 0.90 mmol) was dissolved in ethanol (8 mL), then D-(+)-glucono-1,5-lactone (161 mg, 0.90 mmol) was added, and the whole was heated and refluxed for 3 hours. After cooling, the reaction mixture was concentrated under reduced pressure. The obtained residue was purified by silica gel column chromatography (silica gel 6 g, methanol: methylene chloride = 0:100 to 1:50 to 1:30) to give a long chain glycyl polyol of Formula (5) (116 mg, 0.22 mmol, yield 25%) as a white crystal.

¹H NMR (500 MHz, DMSO-d6, δ ppm): 7.90 (1H, d, J=7.3 Hz, ¹NH Ala), 5.39 (1H, d, J=5.5 Hz, -OH), 5.38-5.3 (2H, m, -CH=CH-), 4.53 (1H, d, J=5.2 Hz, -OH), 4.44 (1H, d, J=5.8 Hz, -OH), 4.38-4.25 (3H, m, -OH, -CH₂-OH, α-CH Ala), 4.10-3.98 (3H, m, -CH(OH)-, -CH₂O-), 3.89 (1H, m, -CH(OH)-), 3.58 (1H, m, -CH₂-OH), 3.52-3.44 (2H, m, -CH(OH)- × 2), 2.0-1.9 (4H, m, -CH₂-CH=CH-CH₂-), 1.60-1.52 (2H, m, -CH₂-), 1.34-1.20 (25H, m, -CH₂-, CH₃ Ala), 0.85 (3H, t, J=7.1 Hz, CH₃).
*Where a signal for 1H in -CH₂-OH was included in a water peak at 3.3 ppm.
FT-MS +m/z calc. for C27H52O8N1 [M+H]+ 518.36929, found 518.3699.

Synthetic Example 6 Synthesis of Long Chain Glycyl Polyol of Formula (6) 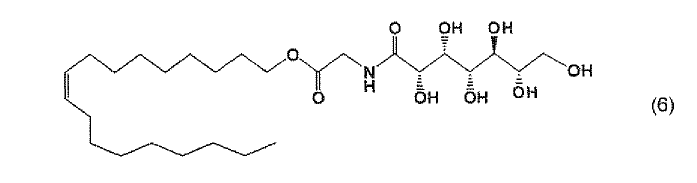 Oleyl alcohol (225 mg, 0.84 mmol) and glycine (63 mg, 0.84 mmol) were suspended in toluene (3 mL), then p-toluenesulfonic acid monohydrate (211 mg, 1.11 mmol) was added, and the whole was heated and refluxed for 5 hours. After cooling, the reaction mixture was diluted with methylene chloride, and successively washed with a saturated aqueous sodium hydrogen carbonate solution and a saturated salt solution. Next, the organic phase was dried over sodium sulfate, then filtered, and concentrated under reduced pressure. The obtained residue was purified by silica gel column chromatography (silica gel 2 g, methanol : methylene chloride =0:100 to 1:30) to give a target compound (219 mg, 0.67 mmol, yield 80%) as a brown liquid.
The compound obtained as described-above (219 mg, 0.67 mmol) was dissolved in ethanol (5 mL), then D-glucoheptono-1,4-lactone (140 mg, 0.67 mmol) was added, and the whole was heated and refluxed for 4 hours. After cooling, the reaction mixture was slowly stirred at room temperature overnight. The resulting crystal was filtered, then washed with ethanol, and dried to give a long chain glycyl polyol of Formula (6) (176 mg, 0.33 mmol, yield 49%) as a white crystal.

¹H NMR (500 MHz, DMSO-d6, δ ppm): 8.23 (1H, t, J=6.1 Hz, ¹NH Gly), 5.74 (1H, d, J=6.4 Hz, -OH), 5.4-5.3 (2H, m, -CH-CH-), 4.79 (1H, d, J=4.3 Hz, -OH), 4.46 (1H, d, J=5.5 Hz, -OH), 4.44 (1H, d, J=5.2 Hz, -OH), 4.35 (1H, d, J=6.7 Hz, -OH), 4.32 (1H, t, J=5.8 Hz, -CH₂-OH), 4.06-3.98 (3H, m, -CH₂O- -CH(OH)-), 3.92-3.82 (3H, m, -CH(OH)-, α-CH × 2 Gly), 3.71 (1H, m, -CH(OH)-), 3.57 (1H, m, -CH₂-OH), 3.52-3.44 (2H, m, -CH(OH)- × 2),2.0-1.9 (4H, m, -CH₂-CH=CH-CH₂-), 1.60-1.52 (2H, m, -CH₂-), 1.34-1.20 (22H, m, -CH2-), 0.85 (3H, t, J=7.1 Hz, CH₃).
*Where a signal for 1H in -CH₂-OH was included in a water peak at 3.3 ppm.
FT-MS +m/z calc. for C27H52O9N1 [M+H]+ 534.36421, found 534.3646.

### Example 1: Evaluation of Gelation with Pure Water

Each long chain glycyl polyol synthesized in Synthetic Example 1 to Synthetic Example 6 was placed in a screw tube (Maruemu No. 1, manufactured by Maruemu Corp.), and ultrapure water (manufactured by Kurita Water Industries Ltd.) was added so that the solution would have a long chain glycyl polyol concentration of 1%, 2%, or 5% (w/v). The solution was heated with a constant temperature heat block (manufactured by Nippon Genetics Co., Ltd.) at 100°C, and then allowed at room temperature for 24 hours. A state where the solution had no flowability and did not run off even when the screw tube was placed in reverse was determined as "gelation (o)". The obtained results are shown in Table 1.

The long chain glycyl polyol of Formula (1) prepared in Synthetic Example 1 formed a sol at concentrations of 1% and 2% (w/v), but was observed to form a gel at a concentration of 5% (w/v).
The long chain glycyl polyol of Formula (2) prepared in Synthetic Example 2 formed a sol at a concentrations of I % (w/v), but was observed to form a gel at a concentration of 2% (w/v).
The long chain glycyl polyol of Formula (6) prepared in Synthetic Example 6 was observed to form a gel at a concentration of 2% (w/v).

**Table 1 Evaluation of Gelation with Pure Water as Medium**

| | Long chain glycyl polyol | Compound concentration (w/v, %) | Gelation |
|---|---|---|---|
| Synthetic Example 1 | (1) | 1 | x |
| Synthetic Example 1 | (1) | 2 | x |
| Synthetic Example 1 | (1) | 5 | ○ |
| Synthetic Example 2 | (2) | 1 | x |
| Synthetic Example 2 | (2) | 2 | ○ |
| Synthetic Example 3 | (3) | 2 | x |
| Synthetic Example 3 | (3) | 5 | x |
| Synthetic Example 4 | (4) | 1 | x |
| Synthetic Example 4 | (4) | 2 | x |
| Synthetic Example 4 | (4) | 5 | x |
| Synthetic Example 5 | (5) | 2 | x |
| Synthetic Example 5 | (5) | 5 | x |
| Synthetic Example 6 | (6) | 1 | x |
| Synthetic Example 6 | (6) | 2 | ○ |

### Example 2: Evaluation of Gelation with Polyethylene Glycol 400 (PEG 400)

Each long chain glycyl polyol synthesized in Synthetic Example 1 to Synthetic Example 6 was placed in a screw tube (Maruemu No. 1, manufactured by (Maruemu Corp.), and PEG 400(manufactured by Wako Pure Chemical Industries, Ltd.) was added so that the solution would have a long chain glycyl polyol concentration of 0.5%, 1%, 2% or 5% (w/v). The solution was heated with a constant temperature heat block (manufactured by Nippon Genetics Co., Ltd.) at 100°C, and then allowed at room temperature for 24 hours. A state where the solution had no flowability and did not run off even when the screw tube was placed in reverse was determined as "gelation (o)". The obtained results are shown in Table 1.

The long chain glycyl polyol of Formula (1) prepared in Synthetic Example 1 formed a sol at a concentration of 0.5% (w/v), but was observed to form a gel at concentrations of 1% and 2% (w/v).
The long chain glycyl polyol of Formula (2) prepared in Synthetic Example 2 was observed to form a gel at a concentration of 5% (w/v).
The long chain glycyl polyol of Formula (4) prepared in Synthetic Example 4 was observed to form a gel at a concentration of 2% (w/v), and the long chain glycyl polyol of Formula (5) prepared in Synthetic Example 5 was observed to form a gel at a concentration of 5% (w/v).

**Table 2 Evaluation of Gelation with PEG 400 as Medium**

| | Long chain glycyl polyol | Compound concentration (w/v, %) | Gelation |
|---|---|---|---|
| Synthetic Example 1 | (1) | 0.5 | x |
| Synthetic Example 1 | (1) | 1 | ○ |
| Synthetic Example 1 | (1) | 2 | ○ |
| Synthetic Example 2 | (2) | 2 | x |
| Synthetic Example 2 | (2) | 5 | ○ |
| Synthetic Example 3 | (3) | 2 | x |
| Synthetic Example 3 | (3) | 5 | x |
| Synthetic Example 4 | (4) | 1 | x |
| Synthetic Example 4 | (4) | 2 | ○ |
| Synthetic Example 4 | (4) | 5 | x |
| Synthetic Example 5 | (5) | 2 | x |
| Synthetic Example 5 | (5) | 5 | ○ |
| Synthetic Example 6 | (6) | 2 | x |
| Synthetic Example 6 | (7) | 5 | x |

### Example 3: Evaluation of Gelation with Olive Oil, Ethanol, or Aqueous Solution (pH = 8)

The long chain glycyl polyol of Formula (1) synthesized in Synthetic Example 1 was placed in a screw tube (Maruemu No. 1, manufactured by Maruemu Corp.), and olive oil (manufactured by Wako Pure Chemical Industries, Ltd.), ethanol, or an aqueous solution (a 6N sodium hydroxide (manufactured by JUNSEI CHEMICAL CO., LTD.) was diluted with water to adjust to pH = 8) was added so that the solution would have a concentration of 0.5% or 1% (w/v). The solution was heated with a constant temperature heat block (manufactured by Nippon Genetics Co., Ltd.) at 100°C, and then allowed at room temperature for 2 to 24 hours. A state where the solution had no flowability and did not run off even when the screw tube was placed in reverse was determined as "gelation (o)". The obtained results are shown in Table 3.

**Table 3 Evaluation of Gelation with Olive Oil, Ethanol, or Aqueous Solution (pH = 8) as Medium**

| | Compound | | Olive oil | Ethanol | Aqueous solution (pH 8) |
|---|---|---|---|---|---|
| | Long chain glycyl polyol | Compound concentration (w/v, %) | Gelation | Gelation | Gelation |
| Synthetic Example 1 | (1) | 0.5 | x | x | x |
| Synthetic Example 1 | (1) | 1.0 | ○ | ○ | ○ |

The long chain glycyl polyol of Formula (1) prepared in Synthetic Example 1 was observed to form a gel at a concentration of 1% (w/v) with olive oil, ethanol, and the aqueous solution (pH = 8) as the medium.

### Example 4: Evaluation of Gelation with Ethanol or Aqueous Solution (pH = 8)

The long chain glycyl polyol of Formula (2) synthesized in Synthetic Example 2 was placed in a screw tube (Maruemu No. 1, manufactured by Maruemu Corp.), and ethanol or an aqueous solution (a 6N sodium hydroxide (manufactured by JUNSEI CHEMICAL CO., LTD.) was diluted with water to adjust to pH = 8) was added so that the solution would have a concentration of 2% or 5% (w/v). The solution was heated with a constant temperature heat block (manufactured by Nippon Genetics Co., Ltd.) at 100°C, and then allowed at room temperature for 2 to 24 hours. A state where the solution had no flowability and did not run off even when the screw tube was placed in reverse was determined as "gelation (o)". The obtained results are shown in Table 4.

**Table 4 Evaluation of Gelation with Ethanol or Aqueous Solution (pH = 8) as Medium**

| | Compound | | Ethanol | Aqueous solution (pH 8) |
|---|---|---|---|---|
| | Long chain glycyl polyol | Compound concentration (w/v, %) | Gelation | Gelation |
| Synthetic Example 2 | (2) | 2.0 | x | ○ |
| Synthetic Example 2 | (2) | 5.0 | ○ | ○ |

The long chain glycyl polyol of Formula (2) prepared in Synthetic Example 2 was observed to form a gel at a concentration of 5% (w/v) with ethanol or the aqueous solution (pH = 8) as the medium, and was further observed to form a gel at a concentration oaf 2% (w/v) with the aqueous solution (pH = 8) as the medium.

As shown in the results above, the long chain glycyl polyol of Formula (1) prepared in Synthetic Example 1 and the long chain glycyl polyol of Formula (2) prepared in Synthetic Example 2 were able to form a gel with each of water, PEG 400, ethanol, and the aqueous solution (pH = 8). The long chain glycyl polyol of Formula (1) prepared in Synthetic Example 1 was able to form a gel even with olive oil as the medium.

### INDUSTRIAL APPLICABILITY

The gelator of the present invention and the gel obtained from the gelator can stably keep a gel structure in a wide pH range from an acidic region to an alkaline region, especially in a neutral condition, and have very high biocompatibility. Therefore, they are preferably used as various functional materials.
For example, from the viewpoint of the application in a wide pH range, they are preferably used for detergents (for example, for medical use, home use, and industrial use), sol-gel forming agents (for cosmetics and other commodities), gelators for stabilizing pigment, food additives (for example, for acidic foods, alkaline foods, and neutral foods), and the like.
Furthermore, in a neutral region, they can be used for biological and biochemical materials, for example, as cell culture substrates and base materials for skin. In an acidic region, they can be used as base materials for pharmaceutical products such as gastric acid-secretion inhibitors, enteric coated preparations, and biodegradable anti-metabolic syndrome preparations by feeling of fullness, as stabilizers and additives for the production of sour milk beverages containing pectin and the like, for improvement of alkali soils, and the like.
Furthermore, in an alkaline region, they can be used as stabilizers and additives for the production of alkaline beverages and milk beverages, for catalytic reactions using various alkaline enzymes (such as alkaline protease, alkaline cellulase, alkaline amylase, alkaline xylase, and alkaline pectate lyase), for industrial use of alkalophilic bacteria, as gelators used for alkaline batteries, for improvement of acidic soils, and as base materials, reaction additives, and accelerators in various industrial applications such as bioreactors, detergent and soap, cosmetics, drug discovery, and analysis and test.

### BRIEF DESCRIPTION OF THE DRAWINGS

[FIG. 1] FIG. 1 is a schematic diagram showing self-assembling of a gelator when it is poured into an aqueous medium and the subsequent gelation.

## Claims

1. A gelator **characterized by** comprising an aliphatic oxyglycyl polyol of Formula (1): 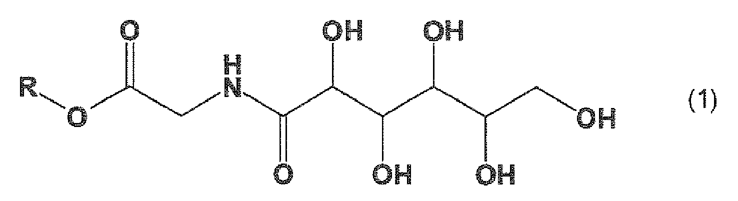 (where R is a C₁₈₋₂₀ saturated aliphatic group or a C₁₈₋₂₀ unsaturated aliphatic group having one double bond) or a pharmaceutically usable salt thereof.

2. The gelator according to claim 1, wherein
R is a C₁₈ saturated aliphatic group or a C₁₈ unsaturated aliphatic group having one double bond.

3. The gelator according to claim 1, wherein
R is a stearyl group or an oleyl group.

4. A self-assembly formed by self-assembling of the gelator as claimed in any one of claims 1 to 3.

5. A gel comprising:
the gelator as claimed in any one of claims 1 to 3 or the self-assembly as claimed in claim 4; and
water, an alcohol, an aqueous solution, an alcohol solution, a hydrophilic organic solution, or a hydrophobic organic solution.

6. The gel according to claim 5, wherein
the alcohol solution is an aqueous solution of at least one alcohol selected from a group consisting of methanol, ethanol, 2-propanol, and i-butanol.

7. The gel according to claim 5, wherein
the hydrophilic organic solution is a solution of at least one hydrophilic organic solvent selected from a group consisting of acetone and dioxane.

8. The gel according to claim 5, wherein
the aqueous solution is an aqueous solution dissolving at least one inorganic salt selected from a group consisting of inorganic carbonates, inorganic sulfates, inorganic phosphates, and inorganic hydrogen phosphates or at least one organic salt selected from a group consisting of organic amine hydrochlorides and organic amine acetates.

9. The gel according to claim 8, wherein
the inorganic salt is at least one inorganic salt selected from a group consisting of calcium carbonate, sodium carbonate, potassium carbonate, sodium sulfate, potassium sulfate, magnesium sulfate, potassium phosphate, sodium phosphate, disodium hydrogen phosphate, and sodium dihydrogen phosphate, and
the organic salt is at least one organic salt selected from a group consisting of ethylenediamine hydrochloride, ethylenediaminetetraacetate, and tris(hydroxymethyl)aminomethane hydrochloride.

10. The gel according to claim 5, wherein
the hydrophobic organic solution is a solution of at least one hydrophobic organic solvent selected from a group consisting of vegetable oils, esters, and hydrocarbons.
